# EUROPEAN PATENT SPECIFICATION

(11) **EP 2 563 498 B1**
(45) Date of publication and mention of the grant of the patent: **24.10.2018**
(21) Application number: 11775382.2
(22) Date of filing: 29.04.2011
(51) Int. Cl.: B01D 53/14, B01D 53/62, C10L 3/10

(54) **BIOGAS UPGRADING**
BIOGASVEREDELUNG
PURIFICATION DE BIOGAZ

(30) Priority: 30.04.2010 US 282963 P; 30.04.2010 SE 1050431
(43) Date of publication of application: 06.03.2013
(73) Proprietor: Purac Puregas AB, 392 39 Kalmar (SE)
(72) Inventor: Karlsson, Lars-Evert, S-380 31 Läckeby (SE)
(74) Representative: AWA Sweden AB
(86) International application number: PCT/SE2011/050539
(87) International publication number: WO 2011/136733

(56) References cited:
- EP-A1- 2 163 293
- DE-A1- 10 325 358
- US-A- 3 659 401
- US-A1- 2010 024 647
- US-B2- 7 276 153

## Description

### Technical Field of the Invention

The present invention relates to a method for upgrading of biogas containing carbon dioxide and a plant for upgrading of biogas containing carbon dioxide as well as uses of the method and the plant.

### Background Art

Biogas production has rendered an increased interest during the last years due to its potential use as an environmental friendly energy source. Biogas is usually produced by anaerobic digestion of biological material, such as municipal waste, energy crops and manure. The main components of the obtained biogas are methane and carbon dioxide. Methane is the component of the biogas having the highest energy value and thus the biogas is frequently upgraded by removing carbon dioxide, which yields a biogas having a higher energy value.

Upgrading of biogas may be achieved by absorption of carbon dioxide from the biogas by means of an absorption liquid. In order to reduce the environmental load and to reduce the costs, the absorption liquid may be recovered by desorption of carbon dioxide from the absorption liquid. The desorption of carbon dioxide may be achieved by heating the absorption liquid to its boiling point. The heating is usually achieved using steam or superheated water also known as subcritical water and pressurized hot water, i.e. liquid water under pressure at temperatures between the usual boiling point (100°C) and the critical temperature (374°C).

WO 2008/034473 discloses a method of separating methane and carbon dioxide from biogas, wherein carbon dioxide present in the biogas is bound in the wash liquid by chemosorption in an absorption column. Thereafter, carbon dioxide bound in the wash liquid is removed by desorption at a pressure of 2 to 30 bar and a temperature of at least 120°C.

US 3,659,401 discloses methods for elimination of impurities from gas mixtures by an absorption stage carried out at above atmospheric pressure, for absorption of the impurities, and a regenerative stage carried out at a pressure substantially lower than that of the absorption stage, generally at atmosperic pressure.

EP 2 163 293 discloses separation of carbon dioxide from a gas mixture.

DE 103 25 358 discloses a process for deacidifying a fluid stream containing acid gases as impurities.

The energy consumption and thus the costs for the energy are considerable in the known techniques and thus there exists a need for a technique that reduces the energy costs.

### Summary of the Invention

An object of the present invention is to provide a method for upgrading of biogas containing carbon dioxide that reduces the energy costs for upgrading of biogas containing carbon dioxide.

This and further objects are achieved by a method for upgrading of biogas containing carbon dioxide comprising absorption of carbon dioxide from biogas containing carbon dioxide by means of an absorption liquid, wherein the absorption is carried out at a pressure of 1013-2000 hPa and desorption of carbon dioxide from the absorption liquid containing carbon dioxide, wherein the desorption is carried out at a pressure of above 400 hPa absolute and below 600 hPa absolute.

The absorption is a chemical absorption.

The absorption liquid is an amine based absorption liquid.

The absorption liquid containing carbon dioxide is heated during the desorption. More specifically, the absorption liquid is recirculated in a closed system, and the absorption liquid containing carbon dioxide is heated using district heating during the desorption to at or above its boiling point.

In one embodiment of the present invention the pressure under which the desorption is carried out is reduced to said pressure.

The method further comprises dewatering of the carbon dioxide released by the desorption using a catch-tank.

Herein is also disclosed a plant for upgrading of biogas containing carbon dioxide which may be used for the present method, which plant comprises an absorption column for absorption of carbon dioxide from biogas containing carbon dioxide by means of an absorption liquid, wherein the absorption is carried out at a pressure of 1013-2000 hPa, and a desorption column for desorption of carbon dioxide from the absorption liquid containing carbon dioxide, wherein the pressure in the desorption column is above 400 hPa absolute and below 600 hPa absolute.

The absorption is a chemical absorption.

The absorption liquid is an amine based absorption liquid.

The plant further comprises a heating device for heating the absorption liquid in the desorption column.

In another embodiment the plant also comprises a pressure reducing device for reducing the pressure in the desorption column to the pressure of above 400 hPa absolute.

The plant further comprises a dewatering device for dewatering the carbon dioxide released by the desorption.

### Brief Description of the Drawings

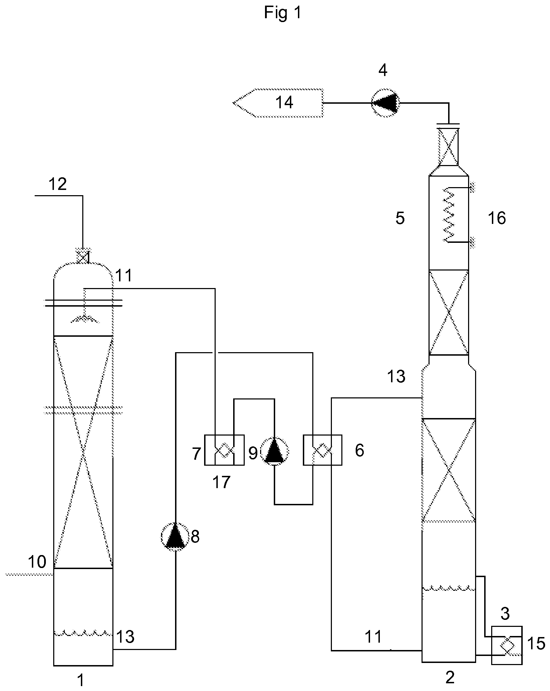

Figure 1 is a schematic drawing of an embodiment of a plant for upgrading of biogas containing carbon dioxide according to the present invention.

### Detailed Description of the Invention

The present invention relates to a method for upgrading of biogas containing carbon dioxide in accordance with claim 1.

In the absorption process, the carbon dioxide is absorbed by the absorption liquid and thus carbon dioxide is removed from the biogas. This increases the methane content in the biogas, which thus has a higher energy value.

In order to enable reuse of the absorption liquid, the absorption liquid is recovered in a desorption process. In the desorption process, the carbon dioxide is released from the absorption liquid. The release of the carbon dioxide from the absorption liquid may be achieved by heating the absorption liquid.

After recovery of the absorption liquid, the absorption liquid may be used once more in the absorption process. The recovery and the reuse of the absorption liquid decreases the environmental load and the costs. After the absorption liquid has been used once more in the absorption process, the recovery of the absorption liquid in the desorption process may be repeated. Therefore, the absorption liquid may be used over and over again. The absorption liquid is recirculated in a closed system. The repeated use and the recirculation of the absorption liquid further decrease the environmental load and the costs.

Since the desorption is carried out at a pressure of above 400 hPa absolute, the desorption is carried out at a lower temperature than when a pressure above atmospheric pressure is used in the desorption. Thus the heating medium used for heating of the absorption liquid during the desorption may have a lower temperature than when a pressure above atmospheric pressure is used in the desorption. The costs for achieving heating of the absorption liquid is thereby decreased, since the costs for obtaining a heating medium having a lower temperature is lower than the costs for obtaining a heating medium having a higher temperature.

In particular, the present invention eliminates the need for steam or superheated water for heating the absorption liquid. The production of steam and superheated water is costly and the present invention therefore considerably reduces the energy costs involved in the desorption. In some embodiments of the present invention, the heating medium used for the heating of the absorption liquid during the desorption may be district heating, which usually is available at a low cost.

The present invention also reduces the energy consumption, since the energy losses are reduced when a lower temperature is utilized. The decreased temperature reduces the energy losses both from the desorption as well as from the obtaining of the heating medium used for heating of the absorption liquid during the desorption. By using a lower temperature, the heat leakage from the desorption process is decreased. This leakage of heat may e.g. be leakage from the vessel in which the desorption is carried out, such as a desorption column, and leakage from the pipes in which the heating medium is transported to the heating device of the desorption process. By using a lower temperature, also the heat leakage from the obtaining of the heating medium having an appropriate temperature for heating the absorption liquid is decreased. This latter leakage of heat may e.g. be leakage from the vessel in which the heating medium having an appropriate temperature is obtained.

In addition, the elimination of the need for steam or superheated water reduces the risks involved in the desorption, since steam and superheated water are associated with e.g. explosion risks and scalding risks. Scalding may cause severe injuries to a person whose body is exposed to the steam or superheated water.

In the method according to the present invention, carbon dioxide is removed from the biogas by an absorption process. In the absorption process the carbon dioxide is absorbed in an absorption liquid. The absorption is carried out at a pressure of 1013-2000 hPa. The absorption is a chemical absorption. In the chemical absorption, carbon dioxide is bound to the absorption liquid.

The absorption liquid is an amine based absorption liquid, e.g. MDEA. The absorption liquid is an aqueous solution of an amine based absorption liquid. The absorption liquid may be an aqueous solution of an amine. The absorption liquid may comprise an amine designed for removal of carbon dioxide.

In the method according to the present invention, the absorption liquid containing carbon dioxide is heated during the desorption. The absorption liquid containing carbon dioxide is heated in order to achieve desorption of carbon dioxide from the absorption liquid containing carbon dioxide. The heating of the absorption liquid is achieved by a heating medium, such as district heating, a medium carrying excess heat from the process of biogas production and/or a medium heated to a suitable level for achievement of desorption of carbon dioxide from the absorption liquid containing carbon dioxide. Naturally, different heating media may be combined in order to achieve sufficient heating of the absorption liquid. For example a medium carrying excess heat from the process of biogas production may not be sufficient and therefore also heat produced specifically for the heating of the absorption liquid may be added.

During the desorption, carbon dioxide is released from the absorption liquid containing carbon dioxide. The release of carbon dioxide from the absorption liquid is dependent on the partial pressure of the carbon dioxide and the pressure at which the desorption is carried out. The release of carbon dioxide from the absorption liquid is accelerated by boiling of the absorption liquid. Thus, in the desorption process, the carbon dioxide may be released by transferring the absorption liquid to or above its boiling point.

In the method according to the present invention, the absorption liquid containing carbon dioxide is heated to or above its boiling point during the desorption.

By carrying out the desorption at a pressure of above 300 hPa absolute, the boiling point of the absorption liquid is decreased.

In case of a chemical absorption, the bond between the carbon dioxide and the absorption liquid is broken before the carbon dioxide is released from the absorption liquid. This breaking of the bond between the carbon dioxide and the absorption liquid may be achieved by heating the absorption liquid containing the carbon dioxide.

The choice of a pressure of above 300 hPa absolute during the desorption is dependent on the used absorption liquid, such as the boiling point of the used absorption liquid. In case of a chemical absorption, the choice of the pressure of above 300 hPa absolute is also dependent on the energy consumed for breaking the bond between carbon dioxide and the absorption liquid. Also the availability of heating medium having a specific temperature affects the choice of the pressure of above 300 hPa absolute. At some sites, heating medium of a specific temperature may be available, e.g. as excess heat in the process of biogas production. At some sites, district heating having a specific temperature is available. The pressure of above 300 hPa absolute is preferable chosen as a pressure somewhat below the pressure at which the used absorption liquid boils at the temperature of the available heating medium.

In some embodiments of the method according to the present invention, the absorption liquid containing carbon dioxide is preheated before desorption of carbon dioxide from the absorption liquid containing carbon dioxide by heat exchanging with the clean absorption liquid from the desorption.

In some embodiments of the method according to the present invention, the clean absorption liquid from the desorption is cooled before absorption of carbon dioxide from biogas containing carbon dioxide by heat exchanging with a cooling medium.

In some embodiments of the method according to the present invention, the method comprises reduction of the pressure under which the desorption is carried out to said pressure of above 400 hPa absolute. The pressure is preferably reduced to a pressure somewhat below a pressure sufficiently low to allow boiling of the absorption liquid at the temperature of the available heating medium, since naturally also the reduction of the pressure consumes energy and unnecessary reduction of the pressure causes unnecessary costs. The pressure of above 400 hPa absolute may be achieved by means of a vacuum system, such as at least one vacuum pump and/or at least one vacuum fan.

In some embodiments, the desorption is carried out at a pressure above 400 hPa absolute, such as above 500 hPa absolute.

In some embodiments, the desorption is carried out at a pressure below 600 hPa absolute, such as below 500 hPa absolute.

An absolute pressure means a pressure related to a perfect vacuum. Thus, a pressure of 300 hPa absolute is a pressure of 300 hPa above perfect vacuum. Since atmospheric pressure is about 1000 hPa, a pressure of 300 hPa absolute corresponds to a negative pressure of about 700 hPa, i.e. a pressure of about 700 hPa below atmospheric pressure.

In the method according to the present invention, the method further comprises dewatering of the carbon dioxide released by the desorption. By removing water present in the carbon dioxide, the reduction of the pressure is facilitated. Water present in the carbon dioxide may render the reduction of the pressure more difficult, e.g. if vacuum pumps and/or vacuum fans are used for obtaining the pressure below atmospheric pressure. The dewatering is achieved by a catch-tank.

In some embodiments of the method according to the present invention, the absorption is carried out in an absorption column.

In some embodiments of the method according to the present invention, the desorption is carried out in a desorption column.

Herein is also disclosed a plant for upgrading of biogas containing carbon dioxide, which plant comprises an absorption column (1) for absorption of carbon dioxide from biogas containing carbon dioxide by means of an absorption liquid, wherein the absorption is carried out at a pressure of 1013-200 hPa, and a desorption column (2) for desorption of carbon dioxide from the absorption liquid containing carbon dioxide, wherein the pressure in the desorption column (2) is a pressure of above 400 hPa absolute.

The reference signs used in the above paragraph and hereafter refer to figure 1.

The carbon dioxide is absorbed by the absorption liquid in the absorption column. Thus, carbon dioxide is removed from the biogas, which yields biogas having a higher energy value.

The carbon dioxide may be released from the absorption liquid by heating of the absorption liquid in the desorption column. Thereby, reuse of the absorption liquid, e.g. recirculation in a closed system, is enabled.

Since the pressure in the desorption column is above 300 hPa absolute, desorption is achieved at a lower temperature. The energy costs involved in the desorption is thereby reduced, since the temperature of the heating medium used for heating the absorption liquid may be decreased. In particular, the plant according to the present invention eliminates the need for costly steam or superheated water.

By using a pressure of above 300 hPa in the desorption column, the temperature used for heating the absorption liquid may be reduced. Thus, the energy losses are reduced and thereby also the energy consumption.

Also the risks associated with the desorption in the desorption column are reduced due to the elimination of the need for steam or superheated water.

In some embodiments of the present invention, the raw biogas (10) enters the absorption column (1) at the bottom and flows upwards. The absorption liquid (11) enters the absorption column (1) at the top and flows downwards and meets the raw biogas. The absorption takes place as the raw biogas meets the absorption liquid in the counter current flow in the absorption column. The upgraded biogas (12) leaves the absorption column (1) at the top and the carbon dioxide enriched absorption liquid (13) leaves the absorption column (1) at the bottom.

In order to achieve a large contact surface between raw biogas and absorption liquid and thus a more efficient absorption, the absorption column may be filled with surface enlarging packing.

The carbon dioxide enriched absorption liquid (13) is transferred, e.g. pumped, to the stripper column (2) for desorption of the carbon dioxide from the absorption liquid containing carbon dioxide. Carbon dioxide may be released from the absorption liquid by heating the absorption liquid.

In some embodiments, the carbon dioxide enriched absorption liquid (13) enters the desorption column (2) at the top and flows downwards. The absorption liquid is heated, and optionally boiled, in the lower part of the desorption column (2) and the raising gas phase strips carbon dioxide from the absorption liquid. The carbon dioxide (14) leaves the desorption column (2) at the top. Optionally, the carbon dioxide is cooled in a condenser (5), e.g. by means of a cooling medium (16), before leaving the desorption column (2). The absorption liquid (11) leaves the desorption column (2) at the bottom.

In order to increase the efficiency of the desorption, the absorption liquid may flow downwards through surface enlarging packing.

The absorption column and the desorption column may be considered as the main parts of a carbon dioxide removal unit. The desorption column may also be named a stripper column.

The absorption is a chemical absorption.

The absorption liquid is an amine based absorption liquid, e.g. MDEA.

In some embodiments, the plant further comprises a heating device (3) for heating the absorption liquid in the desorption column (2). The heating of the absorption liquid facilitates or enables the desorption of carbon dioxide from the absorption liquid containing carbon dioxide according to above. The heating device (3) may be a heat exchanger, which may be heated by a heating medium (15), such as district heating, a medium carrying excess heat from the process of biogas production and/or a medium heated to a suitable level for achievement of desorption of carbon dioxide from the absorption liquid containing carbon dioxide. Naturally, different heating media may be combined in order to achieve a temperature sufficient for heating the absorption liquid.

As said above, the pressure of above 300 hPa absolute in the desorption column is dependent on the used absorption liquid, such as the boiling point of the absorption liquid, in combination with the availability of heating medium having a specific temperature.

In the plant, the heating device for heating the absorption liquid heats the absorption liquid to or above its boiling point in the desorption column.

In some embodiments of the plant further comprises a heat exchanger (6) for preheating the carbon dioxide enriched absorption liquid (13) before entering the desorption column (2) by means of the clean absorption liquid (11). In some embodiments of the plant, the plant further comprises a pump (8) transporting the carbon dioxide enriched absorption liquid (13) in a pipe system from the absorption column (1) to the desorption column (2).

In some embodiments of the plant, the plant further comprises a heat exchanger (7) for cooling clean absorption liquid (11) before entering the absorption column (1) by means of a cooling medium (17). In some embodiments of the plant according to the present invention, the plant further comprises a pump (9) transporting the clean absorption liquid (11) in a pipe system from the desorption column (2) to the absorption column (1).

In some embodiments of the plant, the plant further comprises a pressure reducing device (4) for reducing the pressure in the desorption column (2) to the pressure of above 400 hPa absolute. The pressure in the desorption column may be decreased by means of a vacuum system, such as at least one vacuum pump and/or at least one vacuum fan. Thus, in some embodiments, the plant comprises a vacuum system, such as at least one vacuum pump and/or at least one vacuum fan for reduction of the pressure in the desorption column to the pressure of above 400 hPa absolute. In particular, at least one vacuum pump may be suitable. The pressure reducing device for reducing the pressure, such as a vacuum system, may be located downstream the biogas flow after desorption in the desorption column.

In some embodiments of the plant, the pressure in the desorption column is above 400 hPa absolute, such as above 500 hPa absolute.

In some embodiments of the plant, the pressure in the desorption column is below 600 hPa absolute, such as below 500 hPa absolute.

The plant further comprises a dewatering device for dewatering the carbon dioxide released by the desorption. The dewatering may be achieved by means of at least one catch-tank. By dewatering the carbon dioxide, by means of a catch-tank, the reduction of the pressure is facilitated, since water present in the carbon dioxide may affect the reduction of the pressure negatively. Water contained in the carbon dioxide may typically negatively affect the functionality of vacuum pumps and/or vacuum fans used for obtaining the pressure below atmospheric pressure.

### Example

CO₂ and H₂S are absorbed from biogas at a pressure of 1013-2000 hPa by letting a gas stream meet a fluid stream in an absorption column provided with surface enlarging packing.

As the biogas reaches the top of the absorption column the amount of CO₂ in the biogas will be reduced to < 0.1 percentage by volume. The temperature in the absorption column typically is about 45-55°C.

As the CO₂ and H₂S have been absorbed in the absorption liquid flowing downwards, the absorption liquid is heated in a heat exchanger with heat from heated absorption liquid which is pumped back to the absorption column. After being heated, typically to about 80-95°C, the absorption liquid flows into a stripper column filled with surface enlarging packing. The pressure in the stripper column typically is about 500 hPa (0.5 bar) absolute.

The liquid in the stripper column releases the gas absorbed in the liquid while the liquid flows downwards over the surface enlarging packing.

The desorbed liquid having a temperature of about 85-90°C thereafter passes a heat exchanger where the majority of its energy is submitted to the absorption liquid before the desorption. The temperature of the liquid at this step drops to about 55°C.

The negative pressure in the stripper column may be accomplished e.g. by way of CO₂ and/or H₂S being removed by vacuum pump or other device reducing the pressure.

## Claims

1. Method for upgrading of biogas containing carbon dioxide comprising
absorption of carbon dioxide from biogas containing carbon dioxide by means of an absorption liquid which is an amine based aqueous solution, wherein the absorption is carried out at a pressure of 1013-2000 hPa, and
desorption of carbon dioxide from the absorption liquid containing carbon dioxide, wherein the desorption is carried out at a pressure of above 400 hPa absolute and below 600 hPa absolute,
wherein the absorption liquid is recirculated in a closed system, and the absorption liquid containing carbon dioxide is heated using district heating during the desorption to at or above its boiling point, and
wherein the carbon dioxide released by the desorption is dewatered using a catch-tank.

## Patentansprüche

1. Verfahren zur Veredelung von Kohlendioxid enthaltendem Biogas, umfassend
Absorption von Kohlendioxid aus Kohlendioxid enthaltendem Biogas mit Hilfe einer Absorptionsflüssigkeit, bei dem es sich um eine wässrige Lösung auf Amin-Basis handelt, wobei die Absorption bei einem Druck von 1013-2000 hPa durchgeführt wird, und
Desorption von Kohlendioxid aus der Kohlendioxid enthaltenden Absorptionsflüssigkeit, wobei die Desorption bei einem Druck von mehr als 400 hPa absolut und unter 600 hPa absolut durchgeführt wird,
wobei die Absorptionsflüssigkeit in einem geschlossenen System rezirkuliert wird und die Kohlendioxid enthaltende Absorptionsflüssigkeit unter Verwendung von Fernheizung während der Desorption auf oder über ihren Siedepunkt erhitzt wird und
wobei das durch die Desorption freigesetzte Kohlendioxid unter Verwendung eines Auffangbehälters entwässert wird.

## Revendications

1. Procédé pour la valorisation de biogaz contenant du dioxyde de carbone comprenant
l'absorption de dioxyde de carbone à partir de biogaz contenant du dioxyde de carbone au moyen d'un liquide d'absorption qui est une solution aqueuse à base d'amine, l'absorption étant effectuée à une pression de 1013-2000 hPa, et
la désorption de dioxyde de carbone hors du liquide d'absorption contenant du dioxyde de carbone, la désorption étant effectuée à une pression absolue au-dessus de 400 hPa et au-dessous de 600 hPa,
dans lequel le liquide d'absorption est amené à recirculer dans un système fermé et le liquide d'absorption contenant du dioxyde de carbone est chauffé à l'aide d'un chauffage urbain pendant la désorption à son point d'ébullition ou au-dessus de celui-ci et
dans lequel le dioxyde de carbone libéré par la désorption est déshydraté à l'aide d'un réservoir collecteur.
